(19) Europäisches Patentamt
European Patent Office
Office européen des brevets

(11) Numéro de publication : **0 121 269**
**B1**

(12) # FASCICULE DE BREVET EUROPÉEN

(45) Date de publication du fascicule du brevet : **27.07.88**

(21) Numéro de dépôt : **84200098.6**

(22) Date de dépôt : **26.01.84**

(51) Int. Cl.⁴ : **A 61 L 2/08**, C 08 L 23/02, C 08 K 5/00

(54) **Procédé pour la stérilisation par irradiation de compositions polyoléfiniques.**

(30) Priorité : **07.02.83 FR 8302005**

(43) Date de publication de la demande : **10.10.84 Bulletin 84/41**

(45) Mention de la délivrance du brevet : **27.07.88 Bulletin 88/30**

(84) Etats contractants désignés : **AT BE CH DE FR GB IT LI NL SE**

(56) Documents cités :
**EP-A- 0 091 152**
**FR-A- 1 485 977**
**FR-A- 2 260 571**
**FR-A- 2 264 002**
**GB-A- 1 050 802**
**GB-A- 1 422 454**
**US-A- 3 248 248**

(73) Titulaire : **SOLVAY & Cie (Société Anonyme)**
**Rue du Prince Albert, 33**
**B-1050 Bruxelles (BE)**

(72) Inventeur : **Blanc, Serge**
**Clos des Trigonelles, 3 (boîte 18)**
**B-1120 Bruxelles (BE)**
Inventeur : **van Asbroeck, Roger**
**Langerodestraat, 15**
**B-3055 Neerijse (BE)**

**EP 0 121 269 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé pour la stérilisation par irradiation de compositions polyoléfiniques. Elle concerne plus particulièrement un procédé pour la stérilisation par irradiation gamma de compositions à base de polymères du propylène.

On sait que toutes les polyoléfines présentent l'inconvénient de se dégrader par suite de phénomènes d'oxydation qui sont accélérés sous l'effet de divers facteurs. Cette dégradation entraîne un accroissement de la coloration et une diminution des propriétés mécaniques des articles façonnés à partir de ces polyoléfines.

L'incorporation, aux polyoléfines, de systèmes stabilisants contenant des antioxydants phénoliques, constitue un moyen conventionnel de s'opposer avec efficacité à l'apparition de ces phénomènes.

Les polyoléfines ont, d'autre part, trouvé récemment une application dans la fabrication d'articles médicaux et d'emballages alimentaires généralement soumis à des traitements de stérilisation par irradiation gamma à des doses habituellement comprises entre $0{,}5.10^4$ et $6.10^4$ Gy (gray) (0,5 à 6 mégarads).

Toutefois, l'irradiation gamma aux doses élevées impliquées par la stérilisation d'articles contenant des polyoléfines entraîne une dégradation rapide de ces dernières, qui n'est pas empêchée par la plupart des systèmes stabilisants conventionnels pourtant efficaces en d'autres circonstances. Au contraire, et fâcheusement, l'incorporation de ces systèmes stabilisants entraîne un accroissement de la coloration des articles irradiés.

On a tenté de pallier ces inconvénients par la recherche de systèmes stabilisants capables de s'opposer à la dégradation rapide des polyoléfines contenues dans les articles irradiés mais, jusqu'à présent, seuls des systèmes stabilisants contenant le (3'5'-di-tert-butyl-4-hydroxyphényl) propionate de n-octadécyle et le tétrakis [méthylène-3-(3'5'-di-tert-butyl-4-hydroxyphényl)] propionate méthane à titre d'antioxydants phénoliques se sont révélés avantageux à cet égard (brevet GB-A-1 422 454 de CHISSO CORPORATION).

La présente invention fournit un procédé pour la stérilisation par irradiation de compositions polyoléfiniques qui n'engendre pas les phénomènes de dégradation mentionnés plus haut.

L'invention concerne à cet effet un procédé pour la stérilisation par irradiation, par une dose stérilisante de radiations à haute énergie telles que des rayons gamma, de composition polyoléfiniques contenant au moins un antioxydant phénolique selon lequel la composition polyoléfinique contient un antioxydant qui est le téréphtalate de 2,2'-méthylène-bis-(4-méthyl-6-tert-butylphénol).

Les compositions polyoléfiniques à stériliser selon l'invention peuvent aussi contenir un ou plusieurs autres additifs, généralement choisis parmi les composés définis ci-après.

Il peut s'agir d'autres antioxydants phénoliques tels que les monophénols alkylés. A titre d'exemple de monophénol alkylé, on peut citer le 2,6-di-tert-butyl-p-crésol.

Il peut s'agir d'autres antioxydants conventionnels secondaires, agissant de façon synergétique avec les antioxydants phénoliques, tels que les thioesters, les phosphites organiques et leurs mélanges.

Des thioesters utilisables sont, d'une part, les esters aliphatiques de l'acide thiodipropionique et, d'autre part, les esters dérivés de polyols aliphatiques et d'acides alkylthiodipropioniques. A titre d'exemples d'esters aliphatiques de l'acide thiodipropionique, on peut citer les dilauryl-, distéaryl- et dimyristyl-thiodipropionates. A titres d'exemples d'esters dérivés de polyols aliphatiques et d'acides alkylthiodipropioniques, on peut citer l'octaméthylène-bis (3-dodécylthiopropionate), le sulfure de bis(éthylène-3-octadécylthiopropionate) et le tétrakis(3-thiododécylpropionate) de pentaérythritol.

Des phosphites organiques utilisables sont, d'une part, les trialkyl- et les trialkylarylphosphites et d'autre part, les diphosphites cycliques dérivés du pentaérythritol. A titre d'exemples de trialkyl- et trialkylarylphosphites, on peut citer les trinonyl-, tri(nonylphényl)- et tri(2,4-di-tert-butyl-5-méthyl) phénylphosphites. A titre d'exemple de diphosphites cycliques dérivés du pentaérythritol, on peut citer le diphosphite de distéarylpentaérythritol.

Parmi tous les antioxydants secondaires énumérés ci-dessus, les esters aliphatiques de l'acide thiodipropionique, les diphosphites cycliques dérivés du pentaérythritol et leurs mélanges sont préférés et, parmi eux, tout particulièrement les mélanges de diphosphite de distéarylpentaérythritol et de dilaurylthiodipropionate.

Les compositions polyoléfiniques à stériliser selon l'invention peuvent encore contenir un composé stabilisant qui est un sel de calcium d'un acide aliphatique monocarboxylique saturé. A titre d'exemples de pareils sels, on peut citer les stéarate, palmitate et 2-éthylhexanoate. Le sel préféré est le stéarate de calcium.

L'antioxydant ou le mélange d'antioxydants ainsi que l'éventuel composé stabilisant sont utilisés aux doses stabilisantes usuelles. Ces doses sont habituellement comprises, pour chacun des additifs, entre 0,001 et 10 % en poids des polymères d'alpha-oléfines entrant dans les compositions, de préférence entre 0,005 et 5 % en poids et plus particulièrement entre 0,01 et 0,5 % en poids.

Outre le polymère, les antioxydants ainsi que l'éventuel composé stabilisant, les compositions stérilisées selon l'invention peuvent contenir d'autres additifs usuels tels que, par exemple, d'autres antioxydants tels que les tétrakisphénols, les amines, des agents renforçants, des pigments, lubrifiants, charges, agents antistatiques, etc.

Les polymères d'alpha-oléfines entrant dans les compositions à stériliser selon l'invention sont des polymères contenant au moins 50 % molaires et de préférence au moins 75 % molaires d'oléfines à insaturation terminale dont la molécule contient de 2 à 18 et de préférence de 2 à 6 atomes de carbone telles que l'éthylène, le propylène, le butène-1, le pentène-1, les méthylbutènes-1, l'hexène-1 et les 3- et 4-méthylpentènes-1. Il s'agit plus particulièrement de polymères contenant des atomes de carbone tertiaire tels que les polymères cristallins fortement isotactiques du butène-1, du 4-méthylpentène-1, et tout particulièrement, du propylène.

Il peut s'agir également de copolymères de ces alpha-oléfines entre elles et/ou avec des dioléfines comprenant de 4 à 18 atomes de carbone, telles que des dioléfines aliphatiques non conjuguées comme par exemple l'hexadiène-1,4 ou telles que des dioléfines alicycliques ayant un pont endocyclique comme le dicyclopentadiène par exemple.

Il peut s'agir enfin de copolymères appelés à blocs qui consistent en des successions de segments de chaîne à longueurs variables, chaque segment étant constitué d'un homopolymère d'une alpha-oléfine ou d'un copolymère statistique comprenant une alpha-oléfine et au moins un comonomère choisi parmi les alpha-oléfines et les dioléfines.

Les meilleurs résultats sont obtenus avec les polymères contenant au moins 50 % en poids et de préférence au moins 75 % en poids de propylène.

Les compositions polyoléfiniques à stériliser selon l'invention peuvent aussi être à base de mélanges de deux ou plusieurs polymères tels que décrits ci-dessus, ainsi que d'autres polymères compatibles avec ces derniers.

Les polymères utilisables peuvent être préparés selon les méthodes connues de polymérisation des alpha-oléfines à basse pression. En particulier, les homopolymères cristallins fortement isotactiques du propylène peuvent être préparés en présence de systèmes catalytiques stéréospécifiques à base de composés organométalliques et de chlorures de titane se trouvant à une valence inférieure à sa valence maximale.

Les additifs peuvent être incorporés au polymère de toute façon connue en soi, par exemple en imprégnant le polymère en poudre ou en granules au moyen d'une solution des antioxydants, ou encore en mélangeant une solution ou une suspension du polymère avec une solution des antioxydants. L'incorporation peut être réalisée par exemple, soit dans un mélangeur ou un broyeur où le solvant est évaporé, soit par malaxage sur des cylindres chauffés ou par extrusion d'un mélange du polymère et de la composition stabilisante.

Selon l'invention, les compositions polyoléfiniques sont stérilisées par irradiation. Cette stérilisation est effectuée le plus commodément après transformation des compositions en articles façonnés. Les radiations à haute énergie utilisables pour la stérilisation peuvent être des rayons gamma fournis par une source de cobalt 60, par exemple. L'irradiation par d'autres rayonnements, tels que des rayons X ou des électrons à haute énergie, peut être également effectuée, pour autant qu'elle assure la stérilisation efficace des compositions. En général, la dose d'irradiation stérilisante est comprise entre $0{,}5.10^4$ et $6.10^4$ Gy, de préférence entre $2.10^4$ et $5.10^4$ Gy.

L'intensité de l'irradiation est généralement telle qu'elle correspond à une dose absorbée comprise entre 2 500 et 20 000 Gy/heure.

L'invention concerne également la stérilisation des articles façonnés à partir des compositions polyoléfiniques définies plus haut. Ces compositions sont aptes à la mise en œuvre par tous les procédés classiques de transformation des matières plastiques en articles façonnés, et plus particulièrement par injection et par extrusion. Des exemples d'articles façonnés stérilisables selon l'invention qui peuvent être obtenus à partir desdites compositions polyoléfiniques sont des articles médicaux tels que des seringues, des forceps, des baxters, des pinces chirurgicales, etc. et des articles d'emballage tels que des sacs, sachets, etc.

Ces articles sont caractérisés à la fois par une excellente résistance au jaunissement et par de bonnes propriétés mécaniques.

L'invention est illustrée par l'exemple ci-après.

Exemple

On prépare une composition polyoléfinique stabilisée par mélange à sec des constituants suivants :

100 parties en poids d'un polymère cristallin du propylène d'indice de fusion (MFI) mesuré à 230 °C sous une charge de 2,16 kg (norme ASTM-D 1238) de 12 g/10 min ;

0,05 partie en poids de téréphtalate de 2,2'-méthylène-bis(4-méthyl-6-tert-butylphénol)

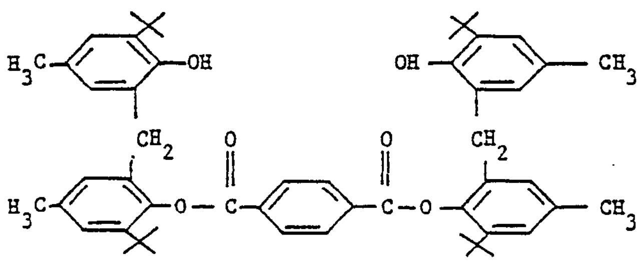

commercialisé par la Société Française d'Organosynthèse sous la dénomination HPM 12 ;

0,05 partie en poids de diphosphite de distéarylpentaérythritol

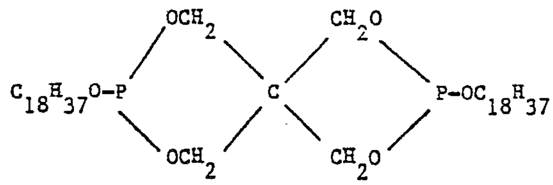

commercialisé sous la marque Weston 618 par la société Borg-Warner ;

0,08 partie en poids de thiodipropionate de dilauryle ;

0,05 partie en poids de stéarate de calcium.

Cette composition stabilisée (1) est transformée par moulage par injection en plaquettes de 2 mm d'épaisseur soumises ensuite à la stérilisation par irradiation au moyen d'une dose de 2 mégarad ($2.10^4$ Gy) de rayons gamma fournis par une source de cobalt 60.

Afin d'apprécier les variations de coloration de ces échantillons provoquées par l'irradiation, les plaquettes irradiée et non irradiée sont soumises à des mesures colorimétriques dans un colorimètre de type HUNTERLAB. A titre de comparaison, on soumet aux mêmes mesures colorimétriques, des plaquettes injectées et irradiées de la même manière que les plaquettes selon l'invention, à partir de compositions polyoléfiniques identiques à celle décrite ci-dessus mais dans lesquelles on a remplacé le téréphtalate de 2,2'-méthylène-bis(4-méthyl-6-tert-butylphénol) respectivement par du (3'5'-di-tert-butyl-4-hydroxyphényl) propionate de n-octadécyle (a) (composition stabilisée (2)) et par du tétrakis [méthylène-3-(3'5'-di-tert-butyl-4-hydroxyphényl) propionate] méthane (b) (composition stabilisée (3)).

Dans le tableau ci-après, on a rassemblé, pour chaque échantillon, avant et après irradiation, la coordonnée colorimétrique b, qui, dans le système colorimétrique L, a, b de Hunter, représente la chromaticité le long de l'axe jaune-bleu, un accroissement de la valeur de cette coordonnée (+ Δb) indiquant un jaunissement (norme ASTM-D2244-79).

Tableau

| Plaquette injectée à partir de la composition | (1) (invention) | (2) (comparaison) | (3) (comparaison) |
|---|---|---|---|
| Coordonnée colorimétrique b de Hunter avant irradiation | 3,3 | 3,6 | 3,5 |
| après irradiation | 4,9 | 4,4 | 5,9 |
| + b | 1,6 | 0,8 | 2,4 |

Les résultats rassemblés dans ce tableau permettent donc de conclure que les compositions irradiées selon l'invention (1) résistent pratiquement aussi bien à la dégradation (jaunissement) que les compositions irradiées (2) (3) contenant les antioxydants phénoliques (a) et (b).

**Revendications**

1. Procédé pour la stérilisation par irradiation, par une dose stérilisante de radiations à haute énergie telles que des rayons gamma, de compositions polyoléfiniques contenant au moins un antioxydant phénolique, caractérisé en ce que la composition polyoléfinique contient le téréphtalate de 2,2'-méthylène-bis(4-méthyl-6-tert-butylphénol).

2. Procédé selon la revendication 1 caractérisé en ce que la composition contient en outre un antioxydant secondaire choisi parmi les thioesters, les phosphites organiques et leurs mélanges.

3. Procédé selon la revendication 2 caractérisé en ce que l'antioxydant secondaire est un mélange de thiodipropionate de dilauryle et de diphosphite de distéarylpentaérythritol.

4. Procédé selon l'une quelconque des revendications précédentes caractérisé en ce que la composition contient en outre un sel de calcium d'un acide aliphatique monocarboxylique saturé.

5. Procédé selon l'une quelconque des revendications précédentes caractérisé en ce que le polymère d'alpha-oléfine entrant dans la composition est un polymère cristallin du propylène.

**Claims**

1. Process for the sterilization by irradiation, with a sterilizing dose of high-energy radiation such as gamma rays, of polyolefinic compositions containing at least one phenolic antioxidant, characterized in that the polyolefinic composition contains 2,2'-methylene-bis(4-methyl-6-tert-butylphenol) terephthalate.

2. Process according to Claim 1, characterized in that the composition also contains a secondary antioxidant chosen from thioesters, organic phosphites and mixtures thereof.

3. Process according to Claim 2, characterized in that the secondary antioxidant is a mixture of dilauryl thiodipropionate and distearylpentaerythritol diphosphite.

4. Process according to any one of the preceding claims, characterized in that the composition also contains a calcium salt of a saturated aliphatic monocarboxylic acid.

5. Process according to any one of the preceding claims, characterized in that the alpha-olefin polymer participating in the composition is a crystalline polymer of propylene.

**Patentansprüche**

1. Verfahren zum Sterilisieren mittels Strahlung durch eine sterilisierende Dosis hochenergetischer Strahlen wie gamma-Strahlen, von Polyolefinzusammensetzungen, die mindestens ein phenolisches Antioxydans enthalten, dadurch gekennzeichnet, daß die polyolefinische Zusammensetzung 2,2'-Methylen-bis-(4-methyl-6-tert-butylphenol)-terephtalat enthält.

2. Verfahren nach Anspruch 1, dadurch gekennzeichnet, daß die Zusammensetzung außerdem ein sekundäres Antioxydans enthält, ausgewählt unter den Thioestern, den organischen Phosphiten und deren Mischungen.

3. Verfahren nach Anspruch 2, dadurch gekennzeichnet, daß das sekundäre Antioxydans eine Mischung von Dilaurylthiodipropionat und Distearylpentaerythritoldiphosphit ist.

4. Verfahren nach einem der vorgehenden Ansprüche, dadurch gekennzeichnet, daß die Zusammensetzung außerdem ein Kalziumsalz einer gesättigten aliphatischen Monocarbonsäure enthält.

5. Verfahren nach einem der vorgehenden Ansprüche, dadurch gekennzeichnet, daß das alpha-Olefinpolymer, welches in die Zusammensetzung eingeht, ein kristallines Propylenpolymer ist.